# EUROPÄISCHE PATENTSCHRIFT

(11) **EP 3 856 887 B1**
(45) Veröffentlichungstag und Bekanntmachung des Hinweises auf die Patenterteilung: **03.09.2025**
(21) Anmeldenummer: 19783227.2
(22) Anmeldetag: 23.09.2019
(51) Int. Cl.: C12M 1/00, C12M 1/12

(54) **BIOREAKTOR UND VERFAHREN ZUR KULTIVIERUNG BIOLOGISCHER ZELLEN AN SUBSTRATFILAMENTEN**
BIOREACTOR AND METHOD FOR CULTIVATING BIOLOGICAL CELLS ON SUBSTRATE FILAMENTS
BIORÉACTEUR ET PROCÉDÉ DE CULTURE DE CELLULES BIOLOGIQUES SUR DES SUBSTRATS FILAMENTEUX

(30) Priorität: 25.09.2018 DE 102018123553
(43) Veröffentlichungstag der Anmeldung: 04.08.2021
(73) Patentinhaber: Fraunhofer-Gesellschaft zur Förderung der angewandten Forschung e.V., 80686 München (DE)
(72) Erfinder: ZIMMERMANN, Heiko, 66280 Sulzbach (DE); NEUBAUER, Julia, 97082 Würzburg (DE); GEPP, Michael, 66280 Sulzbach (DE)
(74) Vertreter: v. Bezold & Partner Patentanwälte - PartG mbB
(86) Internationale Anmeldenummer: PCT/EP2019/075539
(87) Internationale Veröffentlichungsnummer: WO 2020/064639

(56) Entgegenhaltungen:
- EP-A1- 2 298 858
- JP-A- H0 923 876
- US-A1- 2004 029 265
- US-A1- 2015 093 823
- MICHAEL J. LANDRY ET AL: "Layers and Multilayers of Self-Assembled Polymers: Tunable Engineered Extracellular Matrix Coatings for Neural Cell Growth", LANGMUIR, vol. 34, no. 30, 31 July 2018 (2018-07-31), US, pages 8709 - 8730, XP055649412, ISSN: 0743-7463, DOI: 10.1021/acs.langmuir.7b04108
- WENFU ZHENG ET AL: "Precise Control of Cell Adhesion by Combination of Surface Chemistry and Soft Lithography", ADVANCED HEALTHCARE MATERIALS, vol. 2, no. 1, 1 January 2013 (2013-01-01), DE, pages 95 - 108, XP055650653, ISSN: 2192-2640, DOI: 10.1002/adhm.201200104

## Beschreibung

Die Erfindung betrifft einen Bioreaktor zur Kultivierung biologischer Zellen, insbesondere einen Bioreaktor, der Substratfilamente mit einer Adhärenz für die Zellen enthält und für die Kultivierung der Zellen im adhärent gebundenen Zustand eingerichtet ist. Die Erfindung betrifft auch ein Verfahren zur Kultivierung biologischer Zellen, wobei der Bioreaktor verwendet wird. Anwendungen der Erfindung sind z. B. in der Biologie, Biotechnologie, Biochemie und Medizin, insbesondere bei der Kultivierung biologischer Zellen und/oder beim Tissue Engineering gegeben.

Die Kultivierung adhärenter biologischer Zellen auf dem Boden von Zellkulturgefäßen, z. B. Schalen, ist allgemein bekannt. Bei zahlreichen Anwendungen z. B. in der Biotechnologie und Medizin, insbesondere beim Tissue Engineering oder beim Zell-Screening, besteht ein Interesse an der Kultivierung sehr großer Zellmengen. Für diese Anwendungen sind einfache Zellkulturgefäße wegen der beschränkten Effizienz, Skalierbarkeit und Zellausbeute ungeeignet. Daher werden zur Kultivierung adhärenter Zellen, wie z. B. induziert pluripotenter Stammzellen, Kultivierungsoberflächen mit einem hohen Oberfläche-zu-Volumen-Verhältnis (Gesamtfläche der Kultivierungsoberflächen / Volumen des Gefäßes) verwendet, die eine Erhöhung der Effizienz und Zellausbeute ermöglichen. Ein aus der Praxis bekanntes Beispiel hierfür ist die adhärente Suspensionskultur auf Mikro-Carriern oder die Prozessierung der Zellen in Hohlfaser-Bioreaktoren.

Die Zellernte von Mikro-Carriern oder Hohlfasern nach erfolgter Zell-Expansion stellt eine Herausforderung bei der Anwendung der herkömmlichen Techniken dar. Typischerweise erfolgt die Zellernte durch die Behandlung der adhärenten Zellen mit Enzymen, welche die Zellen von den jeweiligen Kultivierungsoberflächen lösen. Von Nachteil ist dabei, dass die Enzyme aufgrund ihrer geringen Spezifität gleichzeitig Membranproteine der Zellen zerstören können. Des Weiteren sind zur Ablösung der Zellen Strömungskräfte im Medium erforderlich, welche zu unerwünschtem Scherstress an den Zellen führen können.

D. C. Watson et al. beschreiben in "Biomaterials" 105 (2016) 195-205 einen Hohlfaser-Bioreaktor mit einem Behälter, in dem Hohlfasern angeordnet sind. Zellen adhärieren an der äußeren Oberfläche der Hohlfasern. Die Hohlfasern werden von einem Kultivierungsmedium durchströmt, das durch Poren in den Wänden der Hohlfasern zu den Zellen gelangt. Die Ernte von Zellprodukten erfolgt durch eine Zufuhr eines flüssigen Mediums in den Behälter und ein Umspülen der Hohlfasern mit dem Medium. Die Technik von D. C. Watson et al. hat eine Beschränkung dahingehend, dass nur die Ernte von Zellprodukten beschrieben wird. Zur Ablösung der Zellen von den Hohlfasern wäre die enzymatische Ablösung erforderlich. Des Weiteren haben die Hohlfasern einen relativ großen Durchmesser, was die Ausbeute der Zellkultivierung beschränkt.

Aus WO 2011/116921 A1 ist eine Kultivierungseinrichtung mit thermoreaktiven Substraten bekannt, die zur adhärenten Kopplung biologischer Zellen angeordnet sind. Diese Kultivierungseinrichtung weist jedoch wegen eines relativ geringen Oberfläche-zu-Volumen-Verhältnisses eine beschränkte Effizienz der Kultivierung bzw. Zellausbeute auf.

M. J. Landry et al. beschreiben in "Langmuir" 2018, 34, 8709-8730, Substrate mit extrazellulären Matrixbeschichtungen aus selbstorganisierten Polymeren für das Wachstum von Nervenzellen.

Die Aufgabe der Erfindung ist es, einen verbesserten Bioreaktor zur Kultivierung biologischer Zellen bereitzustellen, mit dem Nachteile herkömmlicher Techniken vermieden werden. Der Bioreaktor soll insbesondere eine Kultivierung biologischer Zellen mit einer erhöhten Effizienz und Ausbeute und/oder mit einer schonenden Ablösung von Zellen von dem Kultivierungssubstrat ermöglichen. Die Ablösung von Zellen soll insbesondere mit verringertem strömungsmechanischem und/oder chemischem Stress an den Zellen ermöglicht werden. Die Aufgabe der Erfindung ist es auch, ein verbessertes Verfahren zur Prozessierung biologischer Zellen bereitzustellen, das mit dem Bioreaktor ausführbar ist und mit dem Nachteile herkömmlicher Techniken vermieden werden.

Diese Aufgaben werden jeweils durch einen Bioreaktor und ein mit diesem ausgeführtes Verfahren zur Prozessierung biologischer Zellen, insbesondere Zellkultivierung, mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Gesichtspunkt der Erfindung wird die o. g. Aufgabe durch einen Bioreaktor gelöst, der für eine Kultivierung (insbesondere Expansion und/oder Differenzierung) biologischer Zellen eingerichtet ist und einen Behälter (Container, Gehäuse) zur Aufnahme eines Kultivierungsmediums (Nährmediums) und einer Vielzahl von Substratfilamenten (Fasern) umfasst. Die Substratfilamente sind im Behälter voneinander beabstandet und/oder sich zumindest abschnittsweise berührend angeordnet und für eine temporäre (reversible) adhärente Kopplung der biologischen Zellen mit Oberflächen der Substratfilamente eingerichtet. Die Substratfilamente, die starr oder biegsam ausgeführt sein können, stellen feste Kultivierungsoberflächen im Bioreaktor bereit. Die adhärente Kopplung der biologischen Zellen mit Oberflächen der Substratfilamente umfasst eine Bindung der Zellen mit den Oberflächen der Substratfilamente durch eine Zell-Substratoberflächen-Interaktion, insbesondere über Zell-Oberfläche-Bindungsmoleküle.

Gemäß der Erfindung weisen die Substratfilamente innerhalb des Behälters eine veränderliche Oberflächenschicht auf, die zwischen einem Adhärenzzustand (Bindungszustand, Interaktionszustand) und einem Freigabezustand schaltbar ist. Die Oberflächenschicht kann eine Schicht aus einem schaltbaren Material sein, welches eine geschlossene Bedeckung bildet. Alternativ kann die Oberflächenschicht durch eine Monolage oder Sub-Monolage schaltbarer Moleküle gebildet werden. Im Adhärenzzustand ist die Oberfläche der Substratfilamente für eine adhärente Kopplung der Zellen mit der Oberflächenschicht konfiguriert, d. h. die Oberfläche der Substratfilamente ist so gebildet, dass die Zellen mit der Oberfläche Bindungskräfte erzeugen, unter deren Wirkung die Zellen an der Oberfläche fixiert sind. Im Freigabezustand ist die adhärente Kopplung der biologischen Zellen mit der Oberflächenschicht im Vergleich zum Adhärenzzustand verringert, d. h. die Oberfläche der Substratfilamente ist so gebildet, dass die Zellen mit der Oberfläche keine Bindungskräfte erzeugen oder die Bindungskräfte derart verringert sind, dass die Zellen von der Oberfläche freigegeben werden oder unter der Wirkung von Medienbewegungen, z. B. Strömungen des Kultivierungsmediums oder eines Spülmediums, ablösbar sind. Die Oberflächenschicht kann für ein einfaches (irreversibles) oder mehrfaches (reversibles) Schalten eingerichtet sein.

Vorteilhafterweise erlaubt der erfindungsgemäße Bioreaktor durch die Wirkung der schaltbaren Oberflächenschicht eine Ablösung von Zellen von den Substratfilamenten ohne die Einwirkung von Enzymen oder anderen chemischen Substanzen und ohne die Einwirkung von Strömungskräften, welche einen die Zellen verändernden Scherstress ausüben würden. Des Weiteren ermöglichen die Substratfilamente mit schaltbaren Oberflächenschichten im Vergleich zu herkömmlichen Hohlfaser-Bioreaktoren die Verwendung von Substratfilamenten mit einem verringerten Durchmesser und/oder einer erhöhten Packungsdichte, was sich durch ein erhöhtes Oberfläche-zu-Volumen Verhältnis vorteilhaft auf die Effizienz und Ausbeute der Zellkultivierung auswirkt. Auch im Vergleich zu herkömmlichen Techniken unter Verwendung thermoreaktiver Substrate kann eine substantielle Erhöhung des Oberfläche-zu-Volumen-Verhältnisses erzielt werden. In einem Bioreaktor können z. B. mindestens 100, insbesondere mindestens 1000, z. B. 5000 oder mehr Substratfilamente angeordnet sein. Die Substratfilamente haben im Behälter vorzugsweise eine Packungsdichte von mindestens 10 % (Volumenanteil im Behälterinneren), z. B. 35 %, insbesondere 50 % oder mehr.

Gemäß einem zweiten allgemeinen Gesichtspunkt der Erfindung wird die o. g. Aufgabe durch ein Verfahren zur Prozessierung biologischer Zellen in einem erfindungsgemäßen Bioreaktor gelöst, wobei durch Einstellung des Adhärenzzustands der Substratfilamente eine temporäre adhärente Kopplung der biologischen Zellen mit den Substratfilamenten erfolgt. Im Adhärenzzustand erfolgt die Kultivierung (optional mit einer Differenzierung) der temporär adhärent gekoppelten biologischen Zellen. Bei Beendigung der Kultivierung erfolgt die Einstellung des Freigabezustands der Substratfilamente, so dass die biologischen Zellen von den Substratfilamenten abgelöst werden können.

Vorteilhafterweise sind verschiedene Substanzen zur Bereitstellung der schaltbaren Oberflächenschicht verfügbar. Gemäß der Erfindung ist die Oberflächenschicht durch eine Licht-Einwirkung zwischen dem Adhärenzzustand und dem Freigabezustand schaltbar. Das Licht kann Wellenlängen im UV-, VIS- und/oder IR-Spektralbereich enthalten. Gemäß einer optionalen Ausführungsform der Erfindung ist die Oberflächenschicht durch eine Wärme-Einwirkung zwischen dem Adhärenzzustand und dem Freigabezustand schaltbar (Wärme-Einwirkungs-Ausführungsform). Die Licht-Einwirkung und die Wärme-Einwirkung sind kombinierbar, indem z. B. durch Licht-Einwirkung, insbesondere IR-Belichtung, eine Temperaturänderung der Oberflächenschicht erzeugt wird.

Vorzugsweise wird bei der Licht-responsiven Funktionalität der Oberflächenschicht der Adhärenzzustand erzeugt, indem die Oberflächenschicht nicht wirksam belichtet wird, insbesondere unbelichtet ist, und der Freigabezustand durch eine Belichtung der Oberflächenschicht eingestellt. Alternativ können umgekehrt der Adhärenzzustand im belichteten Zustand der Oberflächenschicht und der Freigabezustand im nicht wirksam belichteten, insbesondere unbelichteten, Zustand der Oberflächenschicht eingestellt sein. Vorteilhafterweise kann die Licht-responsive Funktionalität der Oberflächenschicht auf verschiedenen Wirkungen basieren, die insbesondere eine Licht-induzierte Änderung chemischer Eigenschaften der Oberflächenschicht (Aktivierung von Bindungsmolekülen), eine Licht-induzierte Änderung der Temperatur der Oberflächenschicht oder eine Licht-induzierte Bindung oder Freisetzung von Substanzen an oder aus der Oberflächenschicht umfassen.

Gemäß der Erfindung umfassen die Substratfilamente Lichtleiter (oder: Lichtleiterfasern), die jeweils zur Verbindung mit einer Schaltlichtquelleneinrichtung und für eine schaltbare Belichtung der Oberflächenschicht aus den Lichtleitern heraus eingerichtet sind. Vorteilhafterweise wird damit eine gleichmäßige Belichtung der Substratfilamente von innen erzielt. Die Lichtleiter sind aus z. B. Glas oder Kunststoff gebildete Wellenleiter, die kompakt oder hohl, und/oder aus einem einzigen Material oder mehrschichtig gebildet sein können. Jedes Substratfilament kann einen einzigen Lichtleiter oder ein Bündel von Lichtleitern umfassen, wobei der oder die Lichtleiter mit der Oberflächenschicht versehen ist.

Ein Lichtleiter mit der hier beschriebenen, mit Licht zwischen dem Adhärenzzustand und dem Freigabezustand schaltbaren Oberflächenschicht stellt einen unabhängigen Gegenstand der Erfindung dar, für den in der vorliegenden Patentanmeldung Schutz beansprucht wird.

Vorteilhafterweise kann die Schaltlichtquelleneinrichtung eine Vielzahl von separat schaltbaren Lichtquellen umfassen. In diesem Fall kann jedes Substratfilament jeweils mit einer der Lichtquellen optisch gekoppelt sein, wobei die Oberflächenschicht jedes Substratfilaments einzeln durch eine Aktivierung der zugehörigen Lichtquelle zwischen dem Adhärenzzustand und dem Freigabezustand schaltbar ist. Diese Ausführungsform der Erfindung hat den Vorteil, dass selektiv Zellen z. B. für Testzwecke oder zur Lieferung von Zellen in verschiedenen Differenzierungszuständen von den Substratfilamenten freigegeben werden können. Alternativ kann die Schaltlichtquelleneinrichtung mindestens eine Lichtquelle umfassen, mit der alle Substratfilamente gemeinsam optisch gekoppelt sind. In diesem Fall sind alle Substratfilamente gleichzeitig mit der mindestens einen Lichtquelle schaltbar.

Besonders bevorzugt ist die Schaltlichtquelleneinrichtung Teils des Bioreaktors und mit den Lichtleitern der Substratfilamente fest verbunden. Vorteilhafterweise wird damit ein kompakter Aufbau des Bioreaktors erzielt.

Gemäß einer besonders bevorzugten Variante der ersten Ausführungsform der Erfindung sind die Lichtleiter für eine schaltbare Belichtung der Oberflächenschicht mit evaneszenten Wellen eingerichtet, die jeweils aus den Lichtleitern von innen in die Oberflächenschicht dringen. Vorteilhafterweise ermöglicht dies die Verwendung verfügbarer Lichtleiter mit unstrukturierten Oberflächen, insbesondere ohne zusätzliche Maßnahmen zur Lichtauskopplung. Des Weiteren wird die selektive Belichtung einzelner Lichtleiter erleichtert, da die evaneszenten Wellen nicht auf benachbarte Lichtleiter einwirken.

Die Belichtungssteuerung erfolgt alternativ oder zusätzlich durch mindestens eine Lichtquelle, die im Behälter des Bioreaktors angeordnet ist. Damit können vorteilhafterweise die Substratfilamente von ihrer Oberfläche her belichtet werden. Die mindestens eine Lichtquelle kann zum Beispiel neben den Substratfilamenten im Behälter angeordnet sein. Alternativ oder zusätzlich können die Substratfilamente aktive Lichtquellen tragen, welche mit Flüssigkristallen schaltbare Leuchtelemente, Leuchtdioden, insbesondere organische Leuchtdioden (OLED), und/oder Chemolumineszenz-Leuchtelemente umfassen, die jeweils für eine schaltbare Belichtung der Oberflächenschicht eingerichtet sind. Diese Lichtquellen können auf den Oberflächen von Lichtleitern oder anderen, draht- oder bandförmigen Filamenten, z. B. aus Kunststoff oder Glas, angeordnet sein. Vorteilhafterweise ermöglichen die aktiven Lichtquellen im Vergleich zur Belichtung mit evaneszenten Wellen eine erhöhte Belichtungsintensität.

Bei der ersten Ausführungsform der Erfindung kann die Oberflächenschicht gemäß bevorzugten Varianten der Erfindung eine Licht-responsive Hydrogelschicht, insbesondere Alginatschicht, und/oder eine Funktionalisierungsschicht auf der Oberfläche der Substratfilamente umfassen. Alle Substratfilamente im Bioreaktor können die gleiche Oberflächenschicht aufweisen, oder die Substratfilamente können verschiedene Oberflächenschichten aufweisen.

Die Licht-responsive Hydrogelschicht ist eine Schicht aus einem Hydrogel-Polymer, dessen Bindungsfähigkeit für biologische Zellen von der Belichtung abhängt. Diese Funktionalität kann direkt durch das Polymere des Hydrogels (kovalent) und/oder durch eine Kompositbildung der Hydrogelschicht aus einem Hydrogel-Polymer und Licht-responsive Bestandteilen, wie z. B. Micellen, Mikro-Partikel, Nano-Partikel, bereitgestellt werden. Geeignete Hydrogel-Polymere umfassen z. B. Alginat, Gelatine, Polyacrylamid, Gellan-Gum, Hyaluronsäure, oder Polyvinylalkohole oder Hydrogel-Polymere mit ähnlichen Eigenschaften wie diese Beispiele. Licht-responsive Bestandteile können z. B. Vesikeln mit eingebetteten Metall-Nanopartikeln und Wirksubstanzen sein. Bei Belichtung der Oberflächenschicht können die Nanopartikel erwärmt werden, so dass die Vesikeln platzen und die Wirksubstanzen freisetzen, die z. B. für eine Ablösung der Zellen von der Oberflächenschicht ausgelegt sind. Abweichend von der herkömmlichen enzymatischen Ablösung sind die Wirksubstanzen lokal an der Oberflächenschicht wirksam, so dass eine unerwünschte Beeinflussung der Zellen auf ein vernachlässigbares Maß minimiert oder ausgeschlossen werden kann.

Die Funktionalisierungsschicht kann z. B. durch Azobenzene, O-Nitrobenzyl-Gruppen und/oder-Benzonitrile gebildet werden, welche an der Oberfläche der Substratfilamente, insbesondere der Lichtleiter angeordnet sind.

Gemäß einer Ausführungsform der Erfindung ist die Oberflächenschicht in Reaktion auf eine positive oder negative Wärme-Einwirkung (oder: thermische Einwirkung) zwischen dem Adhärenzzustand und dem Freigabezustand schaltbar. Der Adhärenzzustand und der Freigabezustand sind jeweils durch eine Abkühlung und eine Erwärmung (oder umgekehrt) der Substratfilamente einstellbar. Vorteilhafterweise können damit weitere stimulierbare Moleküle zur Bildung der Oberflächenschicht verwendet werden.

Vorzugsweise umfassen die Substratfilamente Hohlfasern, die jeweils zur Verbindung mit einer Temperierungseinrichtung und für eine schaltbare Temperierung (Abkühlung und/oder Erwärmung) der Oberflächenschicht mit einem durch die Hohlfaser strömenden Temperierungsmittel eingerichtet sind. Der Bioreaktor kann insbesondere wie ein Hohlfaser-Bioreaktor aufgebaut sein, wobei jedoch im Unterschied zu diesem die Hohlfasern mit einer Temperatur-responsiven Schicht (z.B. aus dem Polymer pNIPAAM) überzogen sind. Die Hohlfasern können porös und zur Versorgung der Zellen mit einem Kultivierungsmedium ausgelegt sein, wobei die Schaltung der Oberflächenschicht durch eine Temperatureinstellung des Kultivierungsmediums erfolgt. Alternativ können die Hohlfasern undurchlässige Wände aufweisen und von einem Temperierungsmittel, wie z. B. Wasser, durchströmt werden.

Eine Hohlfaser mit der hier beschriebenen, durch eine Temperaturänderung zwischen dem Adhärenzzustand und dem Freigabezustand schaltbaren Oberflächenschicht stellt einen unabhängigen Gegenstand der Erfindung dar, für den in der vorliegenden Patentanmeldung Schutz beansprucht wird.

Bei der Wärme-Einwirkungs-Ausführungsform der Erfindung kann die Belichtungssteuerung durch eine Temperierungseinrichtung erfolgen, die mit allen Hohlfasern gekoppelt ist. Damit können vorteilhafterweise alle Substratfilamente gleichzeitig von innen her temperiert werden. Bevorzugt ist jedoch eine Variante der Erfindung, bei der die Temperierungseinrichtung eine Vielzahl von separat schaltbaren Temperierungselementen umfasst und die Hohlfasern jeweils zur Verbindung mit einem der Temperierungselemente eingerichtet sind. In diesem Fall ist die Oberflächenschicht jeder Hohlfaser einzeln durch eine Aktivierung des zugehörigen Temperierungselements zwischen dem Adhärenzzustand und dem Freigabezustand schaltbar.

Bei der Wärme-Einwirkungs-Ausführungsform der Erfindung ist die Oberflächenschicht vorzugsweise aus einem Temperatur-responsiven Hydrogel gebildet. Beispiele anwendbarer Hydrogele umfassen insbesondere Poly(N-Isopropylacrylamid) (pNIPAAM), Poly-(N-vinylalkylamide), z.B. Poly(N-vinylcaprolactam oder Ko-Polymere, wie z.B. poly(L-lactic acid)-Poly(ethylen-Glycol)-poly(L-lactic acid) (PLLA-PEG-PLLA) triblock-Copolymere, und/oder Poly(ethylene oxide)-poly(propylene oxide)-poly (ethylene oxide) (PEO-PPO-PEO)-Copolymere, oder Temperatur-responsive Hydrogel-Polymere mit ähnlichen Eigenschaften wie diese Beispiele.

Gemäß einem weiteren, bevorzugten Merkmal der Erfindung sind die Substratfilamente biegsame Fasern, die sich im Behälter des Bioreaktors erstrecken. Besonders bevorzugt hat der Behälter eine langgestreckte Form (Rohrform oder Quaderform) mit einer Längsrichtung, wobei die Substratfilamente sich in der Längsrichtung des Behälters erstrecken.

Vorzugsweise weist der Behälter mindestens zwei Fluidikanschlüsse auf, die für eine Durchströmung des Behälters mit einem flüssigen Spülmedium voneinander beabstandet angeordnet sind. Die Fluidikanschlüsse sind mit einem Spülmedium-Reservoir und einer schaltbaren Pumpe verbunden. Das Spülmedium umfasst das Kultivierungsmedium oder eine physiologische Flüssigkeit. Im Freigabezustand ist die adhärente Kopplung der biologischen Zellen mit der Oberflächenschicht so verringert ist, dass die biologischen Zellen unter der Wirkung von Strömungskräften des Spülmediums von den Substratfilamenten trennbar sind. Bei Bereitstellung der langgestreckten Form des Behälters sind die Fluidikanschlüsse vorzugsweise an zueinander entgegengesetzten Enden des Behälters vorgesehen.

Gemäß einem weiteren, bevorzugt vorgesehenen Merkmal der Erfindung weist der Behälter mindestens einen Sensoranschluss auf, der für eine Integration von mindestens einem Sensor in den Bioreaktor eingerichtet ist. Der mindestens eine Sensor umfasst vorzugsweise mindestens eines von einem Temperatur-Sensor, einem pH-Sensor, Glukose-Sensor, Laktat-Sensor, einer Kamera, RAMAN-Sonde. Vorteilhafterweise ermöglicht der mindestens eine Sensor eine Überwachung der Zellkultur während des Betriebes des Bioreaktors.

Weitere Einzelheiten und Vorteile der Erfindung werden im Folgenden unter Bezug auf die beigefügten Zeichnungen beschrieben. Es zeigen:
- Figur 1:: eine schematische Darstellung des Bioreaktors gemäß der Erfindung;
- Figur 2:: weitere Einzelheiten des Bioreaktors gemäß der Erfindung;
- Figur 3:: eine schematische Illustration der Freigabe von biologischen Zellen von der Oberfläche eines Substratfilaments;
- Figur 4:: eine schematische Darstellung der Wärme-Einwirkungs-Ausführungsform des erfindungsgemäßen Bioreaktors; und
- Figur 5:: eine schematische Darstellung des Bioreaktors mit weiteren Komponenten.

Bevorzugte Ausführungsformen der Erfindung werden im Folgenden unter beispielhaftem Bezug auf Bioreaktoren beschrieben, die mit Licht-responsiven oder Wärme-responsiven Substratfilamenten ausgestattet sind. Die Umsetzung der Erfindung ist nicht auf diese Ausführungsformen beschränkt. Alternativ können im Bioreaktor Licht-responsive und Wärme-responsive Substratfilamente kombiniert werden. Des Weiteren ist die Erfindung nicht auf die beispielhaft gezeigte Rohrform des Bioreaktors beschränkt. In Abhängigkeit von der Anwendung der Erfindung können andere Formen des Bioreaktors, wie z. B. Kasten- oder Kugelformen, gewählt werden. Des Weiteren können abweichend von den gezeigten Beispielen aktive Lichtquellen im Behälter des Bioreaktors auf den Substratfilamenten und/oder an einer Innenwand des Behälters vorgesehen sein. Einzelheiten der Erfindung werden insbesondere unter Bezug auf die Gestaltung und Anordnung der Substratfilamente und den Betrieb des Bioreaktors beschrieben. Einzelheiten der Kultivierung biologischer Zellen, insbesondere der Differenzierung adhärent wachsender Stammzellen, werden hier nicht beschrieben, da diese an sich von herkömmlichen Kultivierungsverfahren bekannt sind.

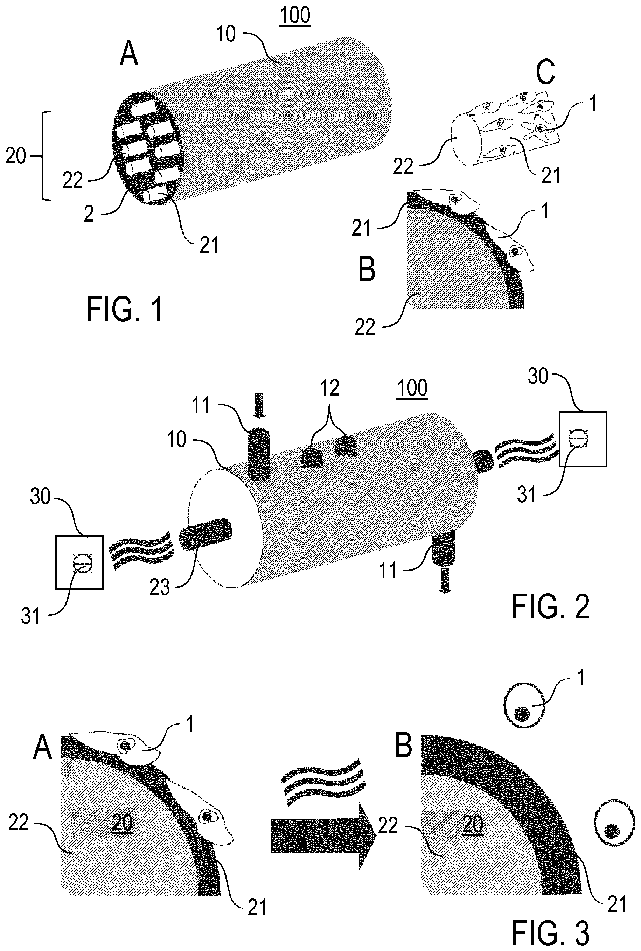

Gemäß der schematischen Teilansicht in Figur 1 umfasst die erste Ausführungsform des erfindungsgemäßen Bioreaktors 100 einen Behälter 10, in dem eine Vielzahl von Substratfilamenten 20 angeordnet sind. Gemäß Figur 1A hat der Behälter 10 die Form eines Hohlzylinders. Der Behälter 10 ist hier offen gezeigt, hat im Betrieb jedoch gemäß der Erfindung eine allseits geschlossene Behälterwand, ggf. mit Fluidik- und Sensoranschlüssen (siehe Figur 2), Fenstern und/oder weiteren Zugriffsöffnungen.

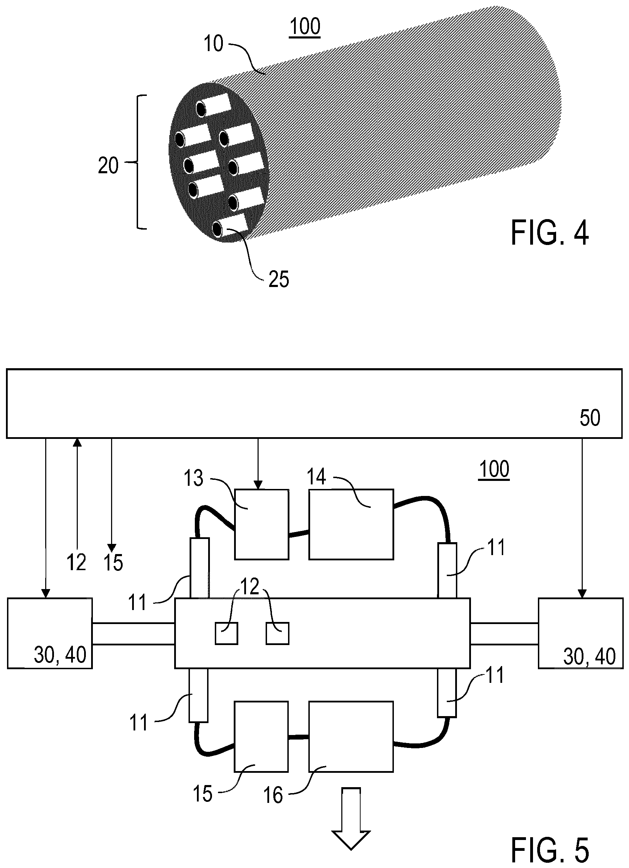

Die Substratfilamente 20 erstrecken sich in axialer Richtung des Behälters 10. Die axiale Länge des Behälters 10 beträgt zum Beispiel 20 cm, und der Durchmesser des Behälters 10 beträgt zum Beispiel 5 cm. Im Behälter 10 sind zum Beispiel 10000 Substratfilamente 20 angeordnet. Der Behälter 10 ist mit einem Kultivierungsmedium 2 gefüllt, welches die Substratfilamente 20 umspült. Vorzugsweise ist eine Durchströmung des Behälters 10 mit dem Kultivierungsmedium 2 vorgesehen (siehe Figuren 2 und 5). Das Kultivierungsmedium 2 umfasst zum Beispiel mTeSR 1-Medium (mTeSR: Produktname).

Jedes Substratfilament 20 umfasst einen Lichtleiter 22 mit einer schaltbaren Oberflächenschicht 21, wie in schematischer Teil-Schnittansicht in Figur 1B und in schematischer Teil-Perspektivansicht in Figur 1C illustriert ist. Der Lichtleiter 21 ist eine kompakte Faser, zum Beispiel aus Glas. Der Durchmesser des Lichtleiters 22 beträgt zum Beispiel 200 µm.

Auf der Oberfläche des Lichtleiters 22 ist die Oberflächenschicht 21, umfassend zum Beispiel ein Licht-responsives Alginat mit angekoppeltem pNIPAAM angeordnet. Die Dicke der Oberflächenschicht 21 beträgt zum Beispiel 0,01 µm. Die Oberflächenschicht 21 ist zwischen einem Adhärenzzustand, in dem biologische Zellen 1 mit der Oberflächenschicht 21 adhärent gekoppelt sind (siehe Figuren 1B und 1C) und einem Freigabezustand schaltbar, in dem die biologischen Zellen 1 von der Oberflächenschicht 21 lösbar sind (siehe Figur 3B).

In Figur 2 sind weitere Einzelheiten der ersten Ausführungsform des erfindungsgemäßen Bioreaktors 100 gezeigt. An den axialen Enden des Behälters 10 treten die Substratfilamente 20 als ein gemeinsames Bündel 23 aus der Behälterwand aus. Eine Schalt-Lichtquelleneinrichtung 30 umfasst zwei Lichtquellen 31, wie zum Beispiel zwei Laser-Quellen, die zur Bestrahlung der freien Enden der Substratfilamente 20 eingerichtet sind. Die Bestrahlung ist durch eine Betätigung der Lichtquellen 31 oder von Shuttern (nicht dargestellt) schaltbar, so dass die Substratfilamente 20 zwischen einem belichteten und einem belichteten Zustand veränderlich sind. Vorzugsweise ist die Oberflächenschicht 21 (siehe Figur 1B) so gestaltet, dass ist im unbelichteten Zustand der Adhärenzzustand und einem belichteten Zustand der Freigabezustand der Substratfilamente 20 eingestellt ist.

Figur 2 zeigt zusätzlich zwei Fluidikanschlüsse 11, die für den dauerhaften oder temporären Anschluss an ein fluidisches System angeordnet und eine Zu- und Abfuhr eines flüssigen Mediums zu und aus dem Behälter 10 ermöglichen. Die Fluidikanschlüsse 11 sind z. B. mit einer Pumpe und einem Medienreservoir verbunden, wie unten unter Bezug auf Figur 5 beschrieben wird. Die Fluidikanschlüsse 11 sind direkt mit dem Innenraum des Behälters 10 zur Aufnahme des Kultivierungsmediums verbunden und erlauben dadurch einen automatisierten Medienwechsel während der Kultivierung, insbesondere Expansion, aber auch die Inokulation/Entnahme von Zellen in Suspension. Des Weiteren ist der Behälter 10 mit zwei Sensoranschlüssen 12 ausgestattet. In den Sensoranschlüssen 12 sind zum Beispiel ein Temperatursensor, ein Glukose-Sensor, ein Laktat-Sensor und/oder ein optischer Sensor, insbesondere eine Kamera, angeordnet.

Figur 3 illustriert schematisch Einzelheiten des Verfahrens zur Zellprozessierung, insbesondere das Umschalten der Substratfilamente 20 vom Adhärenzzustand (Figur 3A) in den Freigabezustand (Figur 3B). Die Adhäsion und Expansion der Zellen 1 erfolgt auf den Substratfilamenten 20, die im Innern des Behälters 10 verlaufen. Die Substratfilamente 20 sind an den Enden gebündelt und verlassen als kompaktes Lichtleiterbündel den Behälter 10 (siehe Figur 2). Kern der Substratfilamente 20 ist das lichtleitende Material der Lichtleiter 22. Das durchgeleitete Licht wird diffus über den gesamten Lichtleiter 22 mit einer bestimmten Wellenlänge emittiert. Das emittierte Licht regt dabei die auf dem Lichtleiter 22 befindliche Oberflächenschicht 21 an und schaltet chemisch oder physikalische Eigenschaften. Beispielsweise kann ein Polymer der Arginylglycylaspartic-Säure (RGD) über den APABA-Linker (4-[(4-aminophenyl)azo]benzocarbonyl), an dem das RGD-Peptid ankonjugiert wurde, durch Licht mit einer Wellenlange von 366 bzw. 450 nm von adhäsiv auf nicht-adhäsiv (und umgekehrt) geschaltet werden. Werden Licht-schaltbare Calciumfänger (Diazo-2) in ionotrope Hydrogele eingebracht, kann durch die Einstrahlung von Licht das Vernetzen aus dem Hydrogel gebunden werden und so das Hydrogel degradiert werden. Adhärente Zellen auf einer solchen Schicht lösen sich dabei von der Kultivierungsfläche und gehen als suspendierte Zellen in den mit Kultivierungsmedium gefüllten Innenraum des Behälters über, aus dem sie über die angeschlossene Fluidik aus dem Bioreaktor transportiert werden können.

Im Adhärenzzustand ist der Lichtleiter 22 unbeleuchtet (keine Bestrahlung der Substratfilamente 20, siehe Figur 2). Die biologischen Zellen 1 sind im Adhärenzzustand adhärent mit der Oberflächenschicht 21 gekoppelt. In diesem Zustand erfolgt die Kultivierung der biologischen Zellen 1 mit dem Kultivierungsmedium 2 (siehe Figur 1A), zum Beispiel für eine Expansion der Zellkultur auf den Substratfilamenten 20 und/oder für eine Differenzierung der Zellen 1. Wenn ein vorbestimmtes Kultivierungsergebnis erzielt ist, werden die Lichtleiter 22 mit den Lichtquellen 31 (siehe Figur 2) belichtet. Die Oberflächenschicht 21 wird in den Freigabezustand umgeschaltet, so dass sich die Zellen 1 von der Oberflächenschicht 21 ablösen. Die Ablösung kann durch eine Bewegung des Kultivierungsmediums in der Umgebung der Substratfilamente gefördert werden.

Figur 4 zeigt schematisch die Wärme-Einwirkungs-Ausführungsform des erfindungsgemäßen Bioreaktors 100. Bei dieser umfassen die Substratfilamente 20 Hohlfasern 25, die sich in axialer Richtung des zylinderförmigen Behälters 10 erstrecken. Die Hohlfasern 25 sind mit einer Pumpe und einem Temperierungsmittel-Reservoir (nicht dargestellt) verbunden. Mit einem Temperierungsmittel, welches die Hohlfasern 25 durchströmt, kann die Temperatur der Hohlfasern 25 eingestellt werden. Beispielsweise kann ein Umschalten zwischen dem Adhärenzzustand bei einer geringeren Temperatur und dem Freigabezustand bei einer höheren Temperatur (oder umgekehrt) vorgesehen sein.

Der Bioreaktor 100 gemäß Figur 4 kann dimensioniert sein, wie oben unter Bezug auf Figur 1 beschrieben ist. Beispielsweise sind 5000 Hohlfasern 25 mit einem Innendurchmesser von 500 µm und einer Oberflächenschicht aus pNIPAAM mit einer Dicke von 0,05 µm vorgesehen.

Figur 5 zeigt eine schematische Übersichtsansicht des Bioreaktors 100 gemäß Ausführungsformen der Erfindung mit dem Behälter 10, in dem die Substratfilamente (nicht dargestellt) angeordnet sind, der Schalt-Lichtquelleneinrichtung 30 oder der Temperierungs-Einrichtung 40 und einer Steuereinrichtung 50. Der Behälter 10 ist im dargestellten Beispiel mit vier Fluidikanschlüssen 11 ausgestattet, die mit einem ersten fluidischen System, umfassend eine erste Pumpe und ein Kultivierungsmedium-Reservoir 14, mit einem zweiten fluidischen System, umfassend eine zweite Pumpe 15 und ein Spülmedium-Reservoir 16 gekoppelt sind. Die Steuereinrichtung 50 ist mit den Komponenten 30, 40 und den Pumpen 13, 15 sowie mit den Sensoren an den Sensoranschlüssen 12 verbunden. Zusätzlich können die fluidischen Systeme mit Sperrelementen, z. B. schaltbaren Ventilen, ausgestattet sein, um wahlweise mit dem Behälter 10 gekoppelt zu werden. Die Sperrelemente können auch mit der Steuereinrichtung 50 steuerbar sein. Alternativ kann nur ein fluidisches System vorgesehen sein, wenn das Spülen der Zellen aus dem Behälter mit dem Kultivierungsmedium erfolgt.

Die Bereitstellung der Steuereinrichtung 50, die zum Beispiel durch einen Computer-Schaltkreis gebildet wird, bietet vorteilhafterweise die Möglichkeit einer Automatisierung des Betriebs des Bioreaktors 100. Es kann ein Regelkreis geschaffen werden, in dem in Abhängigkeit von Signalen der Sensoren an den Sensoranschlüssen 12 oder einem vorgegebenen Kultivierungsprotokoll ein Kultivierungszustand der Zellen erfasst wird. In Abhängigkeit vom Kultivierungszustand kann die Pumpe 13 zur Zufuhr des Kultivierungsmediums gesteuert oder das Umschalten der Substratfilamente vom Adhärenzzustand in den Freigabezustand ausgelöst werden. Des Weiteren kann im Freigabezustand mit der Steuereinrichtung 50 die Pumpe 15 zum Spülen der kultivierten Zellen aus dem Behälters 10 und zur Sammlung der abgelösten Zellen im Spülmedium-Reservoir 16 und zur Abfuhr der Zellen für die weitere Zellprozessierung (siehe Pfeil) gesteuert werden.

## Patentansprüche

1. Bioreaktor (100), der für eine Kultivierung biologischer Zellen (1) eingerichtet ist, umfassend
- einen Behälter (10), der zur Aufnahme eines Kultivierungsmediums (2) eingerichtet ist, und
- eine Vielzahl von Substratfilamenten (20), die im Behälter (10) angeordnet und für eine temporäre adhärente Kopplung der biologischen Zellen (1) mit den Substratfilamenten (20) eingerichtet sind, wobei
- die Substratfilamente (20) mit einer Oberflächenschicht (21) versehen sind, die in Reaktion auf eine Licht-Einwirkung zwischen einem Adhärenzzustand, in dem die biologischen Zellen (1) adhärent an die Oberflächenschicht (21) koppelbar sind, und einem Freigabezustand schaltbar ist, in dem die adhärente Kopplung der biologischen Zellen (1) mit der Oberflächenschicht (21) im Vergleich zum Adhärenzzustand verringert ist,
**dadurch gekennzeichnet, dass**
- der Behälter (10) eine allseits geschlossene Behälterwand aufweist, und
- die Substratfilamente (20) Lichtleiter (22) umfassen, die jeweils zur Verbindung mit einer Schaltlichtquelleneinrichtung (30) und für eine schaltbare Belichtung der Oberflächenschicht (21) aus dem Lichtleiter (22) heraus eingerichtet sind, und/oder im Behälter (10) des Bioreaktors (100) mindestens eine Lichtquelle angeordnet ist, mit der die Substratfilamente (20) von ihrer Oberfläche her belichtet werden können.

2. Bioreaktor gemäß Anspruch 1, bei dem
- die Schaltlichtquelleneinrichtung (30) eine Vielzahl von separat schaltbaren Lichtquellen umfasst, und
- die Substratfilamente (20) jeweils zur Verbindung mit einer der Lichtquellen eingerichtet sind, wobei
- die Oberflächenschicht (21) jedes Substratfilaments (20) einzeln durch eine Aktivierung der zugehörigen Lichtquelle zwischen dem Adhärenzzustand und dem Freigabezustand schaltbar ist.

3. Bioreaktor gemäß einem der vorhergehenden Ansprüche, bei dem
- die Schaltlichtquelleneinrichtung (30) Teils des Bioreaktors (100) und mit den Lichtleitern (22) fest verbunden ist.

4. Bioreaktor gemäß einem der vorhergehenden Ansprüche, bei dem
- die Lichtleiter (22) für eine schaltbare Belichtung der Oberflächenschicht (21) mit evaneszenten Wellen eingerichtet sind, die jeweils aus den Lichtleitern (22) von innen in die Oberflächenschicht (21) dringen.

5. Bioreaktor gemäß einem der vorhergehenden Ansprüche, bei dem
- die Substratfilamente (20) mit Flüssigkristallen schaltbare Leuchtelemente, Leuchtdioden (24), insbesondere organische Leuchtdioden (OLED), und/oder Chemolumineszenz-Leuchtelemente umfassen, die jeweils für eine schaltbare Belichtung der Oberflächenschicht (21) eingerichtet sind.

6. Bioreaktor gemäß einem der vorhergehenden Ansprüche, bei dem die Oberflächenschicht (21) mindestens eines umfasst von
- einer Licht-responsiven Hydrogelschicht, und
- einer Funktionalisierungsschicht auf der Oberfläche der Substratfilamente (20).

7. Bioreaktor gemäß einem der vorhergehenden Ansprüche, bei dem
- die Oberflächenschicht (21) in Reaktion auf eine Wärme-Einwirkung zwischen dem Adhärenzzustand und dem Freigabezustand schaltbar ist.

8. Bioreaktor gemäß einem der vorhergehenden Ansprüche, bei dem
- die Substratfilamente (20) Hohlfasern (25) umfassen, die jeweils zur Verbindung mit einer Temperierungseinrichtung (40) und für eine schaltbare Temperierung der Oberflächenschicht (21) mit einem durch die Hohlfaser (25) strömenden Temperierungsmittel eingerichtet sind.

9. Bioreaktor gemäß Anspruch 8, bei dem
- die Temperierungseinrichtung (40) eine Vielzahl von separat schaltbaren Temperierungselementen umfasst, und
- die Hohlfasern (25) jeweils zur Verbindung mit einem der Temperierungselemente eingerichtet sind, wobei
- die Oberflächenschicht (21) jeder Hohlfaser (25) einzeln durch eine Aktivierung des zugehörigen Temperierungselements zwischen dem Adhärenzzustand und dem Freigabezustand schaltbar ist.

10. Bioreaktor gemäß einem der Ansprüche 7 bis 9, bei dem
- die Oberflächenschicht (21) aus einem Temperatur-responsiven Hydrogel gebildet ist.

11. Bioreaktor gemäß einem der vorhergehenden Ansprüche, bei dem
- die Substratfilamente (20) biegsame Fasern umfassen, die sich im Behälter (10) erstrecken.

12. Bioreaktor gemäß einem der vorhergehenden Ansprüche, bei dem
- der Behälter (10) eine langgestreckte Form aufweist, und
- die Substratfilamente (20) sich in einer Längsrichtung des Behälters (10) erstrecken.

13. Bioreaktor gemäß einem der vorhergehenden Ansprüche, bei dem
- der Behälter (10) mindestens zwei Fluidikanschlüsse (11) aufweist und für eine Durchströmung mit einem flüssigen Spülmedium eingerichtet ist, wobei
- im Freigabezustand die adhärente Kopplung der biologischen Zellen (1) mit der Oberflächenschicht (21) derart verringert ist, dass die biologischen Zellen (1) unter der Wirkung von Strömungskräften des Spülmediums von den Substratfilamenten (20) trennbar sind.

14. Bioreaktor gemäß einem der vorhergehenden Ansprüche, bei dem
- der Behälter (10) mindestens einen Sensoranschluss (12) aufweist, der für eine Integration von mindestens einem Sensor (13) in den Bioreaktor (100) eingerichtet ist.

15. Verfahren zur Prozessierung biologischer Zellen (1) in einem Bioreaktor (100) gemäß einem der vorhergehenden Ansprüche, mit den Schritten:
- Einstellung des Adhärenzzustands der Substratfilamente (20),
- adhärente Kopplung der biologischen Zellen (1) mit den Substratfilamenten (20),
- Kultivierung der adhärent gekoppelten biologischen Zellen (1),
- Einstellung des Freigabezustands der Substratfilamente (20), und
- Ablösung der biologischen Zellen (1) von den Substratfilamenten (20).

## Claims

1. Bioreactor (100) which is adapted for culturing biological cells (1), comprising
- a container (10) which is adapted for receiving a culture medium (2), and
- a plurality of substrate filaments (20) which are arranged in the container (10) and are adapted for temporary adherent coupling of the biological cells (1) to the substrate filaments (20), wherein
- the substrate filaments (20) are provided with a surface layer (21) which, in response to the action of light, can be switched between an adherence state, in which the biological cells (1) can be coupled to the surface layer (21) in an adherent manner, and a release state, in which the adherent coupling of the biological cells (1) to the surface layer (21) is reduced compared to the adherence state,
**characterized in that**
- the container (10) comprises a container wall which is closed on all sides, and
- the substrate filaments (20) comprise light guides (22), which are each adapted for connection to a switching light source device (30) and for switchable illumination of the surface layer (21) out of the light guide (22), and/or at least one light source is arranged in the container (10) of the bioreactor (100), with which the substrate filaments (20) can be illuminated from their surface.

2. Bioreactor according to claim 1, wherein
- the switching light source device (30) comprises a plurality of separately switchable light sources, and
- the substrate filaments (20) are each adapted for connection to one of the light sources, wherein
- the surface layer (21) of each substrate filament (20) is individually switchable between the adherence state and the release state by activating the associated light source.

3. Bioreactor according to one of the preceding claims, wherein
- the switching light source device (30) is part of the bioreactor (100) and is securely connected to the light guides (22).

4. Bioreactor according to one of the preceding claims, wherein
- the light guides (22) are adapted for a switchable illumination of the surface layer (21) with evanescent waves, each of which penetrates from inside the light guides (22) into the surface layer (21).

5. Bioreactor according to one of the preceding claims, wherein
- the substrate filaments (20) are adapted with liquid-crystal switchable light elements, light-emitting diodes (24), in particular organic light-emitting diodes (OLED), and/or chemiluminescent light elements, which are each adapted for switchable illumination of the surface layer (21).

6. Bioreactor according to one of the preceding claims, wherein the surface layer (21) comprises at least one from
- a light-responsive hydrogel layer, and
- a functionalization layer on the surface of the substrate filaments (20).

7. Bioreactor according to one of the preceding claims, wherein
- the surface layer (21) can be switched between the adherence state and the release state in response to the action of heat.

8. Bioreactor according to one of the preceding claims, wherein
- the substrate filaments (20) comprise hollow fibres (25), which are each adapted for connection to a temperature-control device (40) and for switchable temperature control of the surface layer (21) with a temperature-control medium flowing through the hollow fibres (25).

9. Bioreactor according to claim 8, wherein
- the temperature-control device (40) comprises a plurality of separately switchable temperature-control elements, and
- the hollow fibres (25) are each adapted for connection to one of the temperature-control elements, wherein
- the surface layer (21) of each hollow fibre (25) is individually switchable between the adherence state and the release state by activating the associated temperature-control element.

10. Bioreactor according to one of claims 7 to 9, wherein
- the surface layer (21) is formed from a temperature-responsive hydrogel.

11. Bioreactor according to one of the preceding claims, wherein
- the substrate filaments (20) comprise flexible fibres which extend in the container (10).

12. Bioreactor according to one of the preceding claims, wherein
- the container (10) has an elongate form, and
- the substrate filaments (20) extend in a longitudinal direction of the container (10).

13. Bioreactor according to one of the preceding claims, wherein
- the container (10) has at least two fluid connectors (11) and is adapted to be flowed through by a liquid flushing medium, wherein
- in the release state, the adherent coupling of the biological cells (1) to the surface layer (21) is reduced such that the biological cells (1) can be separated from the substrate filaments (20) under the action of the flow forces of the flushing medium.

14. Bioreactor according to one of the preceding claims, wherein
- the container (10) has at least one sensor connector (12) which is adapted for integrating at least one sensor (13) into the bioreactor (100).

15. Method for processing biological cells (1) in a bioreactor (100) according to one of the preceding claims, comprising the steps of:
- setting the adherence state of the substrate filaments (20),
- adherent coupling of the biological cells (1) to the substrate filaments (20),
- culturing the adherently coupled biological cells (1),
- setting the release state of the substrate filaments (20), and
- detaching the biological cells (1) from the substrate filaments (20).

## Revendications

1. Bioréacteur (100), qui est mis au point pour la culture de cellules biologiques (1), comprenant
- un contenant (10), qui est mis au point pour recevoir un milieu de culture (2), et
- une pluralité de substrats filamenteux (20), qui sont disposés dans le contenant (10) et sont mis au point pour coupler par adhérence temporairement les cellules biologiques (1) aux substrats filamenteux (20), dans lequel
- les substrats filamenteux (20) sont pourvus d'une couche de surface (21) qui peut passer, en réponse à une exposition à la lumière, entre un état d'adhérence, dans lequel les cellules biologiques (1) peuvent être couplées par adhérence à la couche de surface (21), et un état de libération, dans lequel le couplage par adhérence des cellules biologiques (1) à la couche de surface (21) est réduit en comparaison avec l'état d'adhérence,
**caractérisé en ce que**
- le contenant (10) présente une paroi de contenant fermée sur tous les côtés, et
- les substrats filamenteux (20) comprennent des guides de lumière (22), qui sont respectivement mis au point pour être reliés à un dispositif de source de lumière de commutation (30) et pour une exposition commutable de la couche de surface (21) à partir du guide de lumière (22), et/ou au moins une source de lumière est disposée dans le contenant (10) du bioréacteur (100) avec laquelle les substrats filamenteux (20) peuvent être exposés à partir de leur surface.

2. Bioréacteur selon la revendication 1, où
- le dispositif de source de lumière de commutation (30) comprend une pluralité de sources de lumière pouvant être commutées séparément, et
- les substrats filamenteux (20) sont respectivement mis au point pour être reliés respectivement à l'une des sources de lumière, dans lequel
- la couche de surface (21) de chaque substrat filamenteux (20) peut être commutée individuellement entre l'état d'adhérence et l'état de libération par une activation de la source de lumière correspondante.

3. Bioréacteur selon l'une quelconque des revendications précédentes, où
- le dispositif de source de lumière de commutation (30) fait partie du bioréacteur (100) et est relié de manière solidaire aux guides de lumière (22).

4. Bioréacteur selon l'une quelconque des revendications précédentes, où
- les guides de lumière (22) sont mis au point pour une exposition commutable de la couche de surface (21) avec des ondes évanescentes qui pénètrent respectivement à partir des guides de lumière (22) de l'intérieur dans la couche de surface (21).

5. Bioréacteur selon l'une quelconque des revendications précédentes, où
- les substrats filamenteux (20) comprennent des éléments lumineux pouvant être commutés à cristaux liquides, des diodes électroluminescentes (24), en particulier des diodes électroluminescentes organiques (OLED), et/ou des éléments lumineux à chimioluminescence, qui sont respectivement mis au point pour une exposition commutable de la couche de surface (21).

6. Bioréacteur selon l'une quelconque des revendications précédentes, où la couche de surface (21) comprend au moins une
- d'une couche d'hydrogel sensible à la lumière, et
- d'une couche de fonctionnalisation sur la surface des substrats filamenteux (20).

7. Bioréacteur selon l'une quelconque des revendications précédentes, où
- la couche de surface (21) peut être commutée entre l'état d'adhérence et l'état de libération en réaction à un effet thermique.

8. Bioréacteur selon l'une quelconque des revendications précédentes, où
- les substrats filamenteux (20) comprennent des fibres creuses (25) qui sont mises au point respectivement pour être reliées à un dispositif de thermorégulation (40) et pour une thermorégulation commutable de la couche de surface (21) avec un moyen de thermorégulation circulant à travers la fibre creuse (25).

9. Bioréacteur selon la revendication 8, où
- le dispositif de thermorégulation (40) comprend une pluralité d'éléments de thermorégulation pouvant être commutés séparément, et
- les fibres creuses (25) sont respectivement mises au point pour être reliées à un des éléments de thermorégulation, dans lequel
- la couche de surface (21) de chaque fibre creuse (25) peut être commutée individuellement entre l'état d'adhérence et l'état de libération par une activation de l'élément de thermorégulation associé.

10. Bioréacteur selon l'une quelconque des revendications 7 à 9, où
- la couche de surface (21) est formée d'un hydrogel sensible à la température.

11. Bioréacteur selon l'une quelconque des revendications précédentes, où
- les substrats filamenteux (20) comprennent des fibres flexibles qui s'étendent dans le contenant (10).

12. Bioréacteur selon l'une quelconque des revendications précédentes, où
- le contenant (10) présente une forme allongée, et
- les substrats filamenteux (20) s'étendent dans une direction longitudinale du contenant (10).

13. Bioréacteur selon l'une quelconque des revendications précédentes, où
- le contenant (10) présente au moins deux raccords fluidiques (11) et est mis au point pour être traversé par un milieu de rinçage liquide, dans lequel
- à l'état libéré, le couplage par adhérence des cellules biologiques (1) à la couche de surface (21) est réduit de telle manière que les cellules biologiques (1) peuvent être séparées des substrats filamenteux (20) sous l'effet de forces d'écoulement du milieu de rinçage.

14. Bioréacteur selon l'une quelconque des revendications précédentes, où
- le contenant (10) présente au moins un raccord de capteur (12) qui est mis au point pour intégrer au moins un capteur (13) dans le bioréacteur (100).

15. Procédé de traitement de cellules biologiques (1) dans un bioréacteur (100) selon l'une quelconque des revendications précédentes, avec les étapes :
- de réglage de l'état d'adhérence des substrats filamenteux (20),
- de couplage par adhérence des cellules biologiques (1) aux substrats filamenteux (20),
- de culture des cellules biologiques (1) couplées par adhérence,
- de réglage de l'état de libération des substrats filamenteux (20), et
- de décollement des cellules biologiques (1) des substrats filamenteux (20).
